# EUROPÄISCHE PATENTSCHRIFT

(11) **EP 2 206 780 B1**
(45) Veröffentlichungstag und Bekanntmachung des Hinweises auf die Patenterteilung: **29.02.2012**
(21) Anmeldenummer: 08172670.5
(22) Anmeldetag: 23.12.2008
(51) Int. Cl.: C12N 15/10, B01L 3/00

(54) **Verfahren und Vorrichtung zur Durchführung einer Nukleinsäurepräparation und/oder Amplifikation**
Method and device for carrying out a nucleic acid preparation and/or amplification
Procédé et dispositif pour la réalisation d'un procédé de préparation d'un acide nucléique et/ou d'amplification

(43) Veröffentlichungstag der Anmeldung: 14.07.2010
(73) Patentinhaber: Qiagen GmbH, 40724 Hilden (DE)
(72) Erfinder: Rohtmann, Thomas, Dr. c/o QIAGEN GmbH, 40724 Hilden (DE); Himmelreich, Ralf, Dr. c/o QIAGEN GmbH, 40724 Hilden (DE); Colpan, Metin c/o QIAGEN GmbH, 40724 Hilden (DE)
(74) Vertreter: Gille Hrabal

(56) Entgegenhaltungen:
- WO-A-94/26414
- WO-A-03/007677
- WO-A-2007/100500
- US-A1- 2008 038 813

## Beschreibung

Die Erfindung betrifft ein Verfahren und eine Vorrichtung zur Durchführung einer Nukleinsäurepräparation und/ oder Amplifikation.

Aus dem Stand der Technik ist bekannt, für die Vorbereitung einer biologischen Probe zunächst den Inhalt einer biologischen Probe zugänglich zu machen (bekannt unter der Bezeichnung "Lyse" oder "Aufschließen"), Bestandteile des freigesetzten Inhalts der biologischen Probe an oder auf einem festen Stütz- oder Trägermaterial selektiv zu binden (bekannt unter der Bezeichnung "Binden"), unerwünschte Bestandteile vom festen Stütz- oder Trägermaterial zu beseitigen (bekannt unter der Bezeichnung "Waschen") und die gewünschten Bestandteile, nämlich Nukleinsäuren anschließend vom festen Stütz- oder Trägermaterial zu lösen (bekannt unter der Bezeichnung "Elution"). Zumindest ein gesuchter Bestandteil, also ein definierter Teil eines DNA- oder RNA-Strangs wird schließlich beispielsweise mittels einer Polymerase-Kettenreaktion (PCR) vervielfältigt, also amplifiziert.

Ein automatisiertes Verfahren für die Vorbereitung einer biologischen Probe, welches die Schritte Lyse, Binden, Waschen, Elution und PCR umfasst, ist der europäischen Patentanmeldung mit dem amtlichen Aktenzeichen EP 08151 152.9 zu entnehmen: Eine biologische Probe wird in einen Behälter gebracht, der einen beispielsweise aus einem Silica-Gel bestehenden Filter aufweist. Unterhalb des Filters ist ein Zu- bzw. Abfluss für Flüssigkeit vorgesehen. Zunächst wird eine biologische Probe insbesondere in diesem Behälter mit einem Lysepuffer aufgeschlossen. Nach dem Aufschließen der Probe wird der Lysepuffer durch den Filter hindurch abgesaugt. Es wird dadurch zugleich erreicht, dass die durch Aufschluss freigesetzte Nukleinsäure an den Filter bindet. Das Binden der Nukleinsäure an den Filter wird durch eine anschließende Zugabe von Ethanol in das Gefäß verstärkt. Mit Hilfe von Waschpuffern wird danach gewaschen, um so Fette, Lipide und Proteine zu entfernen. Nach dem Waschen wird die gebundene Nukleinsäure mit Hilfe von Elutionspuffer von dem Filter gelöst. Der Elutionspuffer wird zusammen mit den nun darin befindlichen Nukleinsäuren einer PCR-Kammer zugeführt, um darin die gewünschte Polymerase-Kettenreaktion durchzuführen.

Aus der europäischen Patentanmeldung mit dem amtlichen Aktenzeichen EP08151152.9 geht weiter hervor, dass Ergebnis der PCR mit Hilfe eines Agarosegels elektrophoretisch aufzutrennen und die aufgetrennten Nukleinsäurefragmente in einem Ethidiumbromid-haltigem Wasserbad einzufärben. Durch Anregung mit UV-Licht werden die eingefärbten Nukleinsäurefragmente sichtbar und so nachgewiesen. Die Bindung erfolgt auf Basis von "chaotrop" Chemie. Es wird dann unter Hoch-Salz (also einer Lösung mit hoher Salzkonzentration) gebunden. Eine Ethanol - Elution erfolgt mit Niedrig-Salz, also einer Lösung mit niedriger Salzkonzentration. Wird dagegen ein Anionenaustauscher eingesetzt, so erfolgt die Bindung unter Niedrig-Salz und Elution unter Hoch-Salz

Aus der Druckschrift WO2008/006500 ist eine Vorrichtung bekannt, mit der Nukleinsäure automatisiert präpariert und amplifiziert werden kann. Die Vorrichtung umfasst eine Mehrzahl an Arbeitsstationen, um eine in einen Wegwerfbehälter hinein pipettierte flüssige biologische Probe wie Plasma und Blut zu präparieren und zu amplifizieren. Der Wegwerfbehälter umfasst verschiedene Kammern, durch die flüssige Probe mit Hilfe von Druckluft transportiert wird. Für die Präparation und Amplifikation benötigte Reagenzien werden zum Teil von der Vorrichtung in den Wegwerfbehölter hineingepumpt und zum Teil befinden sich Reagenzien bzw. feste Substanzen von Anfang an im Wegwerfbehälter. Nicht mehr benötigte Reagenzien können aus dem Wegwerfbehälter in die Vorrichtung zurück transportiert werden.

Zwar ist die Herstellung des hieraus bekannten Wegwerfbehälters bereits relativ kostengünstig im Vergleich zu anderen bekannten Wegwerfbehältern, die für die Durchführung einer Nukleinsäurepräparation und/ oder Amplifikation vorgesehen sind, da aufgrund Vorrichtung mit den verschiedenen Arbeitsstationen ein relativ einfach konstruierter Behälter genügt. Allerdings müssen für die Herstellung des Wegwerfbehälters immer noch sehr teure Spritzgussformen hergestellt werden. Auch die Vorrichtung mit den verschiedenen Arbeitsstationen ist relativ teuer. So kommt der Stand der Technik nicht damit aus, lediglich zu pipettieren. Werden Abfallreagenzien aus dem Wegwerfbehälter entfernt, so ist damit das Risiko einer Kontamination verbunden. Eine Kontamination droht ferner, weil die Substanzen im Wegwerfbehälter nicht stets gegenüber der Umwelt verschlossen sind.

Auf der Internetseite http://www.devicelink.com/ivdt/archlve/04/07/011.html sowie in der Druckschrift US 2004/0161788 A1 wird eine Probenpräparation sowie eine Probenamplifikation beschrieben, die in einem schlauchförmigen Behälter durchgeführt wird. Der schlauchförmige Behälter ist in einzelne Segmente unterteilt. Die einzelnen Segmente sind durch Dichtungen zunächst voneinander flüssigkeitsdicht getrennt. Die Dichtungen können so aufgebrochen werden, dass zwei Segmente miteinander verbunden werden. Zunächst sind Segmente mit Reagenzien gefüllt. Die Probenpräparation und Probenamplifikation wird durchgeführt, indem Segmente zusammengepresst werden, so u. a. Dichtungen aufgebrochen und Segmente geeignet miteinander verbunden werden. Darüberhinaus kann mithilfe von Stößeln eine Unterteilung in Segmente vorgenommen werden, um so das gewünschte Verfahren durchzuführen.

Da der schlauchförmige Behälter vorgefüllt und in Segmente unterteilt werden muss, ist seine Herstellung relativ kostenaufwendig. Nachteilhaft müssen Dichtungen aufgebrochen werden, was Störungen zur Folge haben kann, wenn in fehlerhafter Weise aufgebrochen wird.

Nicht mehr benötigte Reagenzien werden in dem schlauchförmigen Behälter durch entsprechendes Zusammenpressen des Behälters nach oben transportiert und gelangen so in eine Verschlusskappe des Behälters hinein, mit der der Behälter verschlossen wird. Nachteilhaft hieran ist, dass aufgrund von Undichligkeiten zwischen Kappe und Behälter eine Kontamination auftreten kann. Problematisch ist an diesem Stand der Technik feirer, dass u. a. mit Druck der Flüssigkeltransport im Behälter gesteuert wird So werden beispielsweise mittels Druck nicht mehr benötigte Reagenzien in die Verschlusskappe hinein gefördert. Dies erhöht das Risiko, dass aufgrund von Undichtigkeiten die Umgebung kontaminiert w:rd. Es findet beim vorgenannten Stand dar Technik m!t dem schlauchförmigen Behälter kein Druckausgleich mit der Umgebung statt, wodurch das System störanfällig wird.

Die Druckschrift US 2008/0038813 A1 offenbart, Flüssigkeit in einem Schlauch In verschiedene Bereiche durch Zusammenpressen transportieren zu können, umso in gewünschte Regionen eines Automaten die Flüssigkeit transportieren zu können. Eine Probe soll so unterschiedliche Temperaturzonen innerhalb des Automaten durchlaufen.

Die Druckschrift in WO 2007/100500 A2 betrifft ein flexibles, segmentiertes Röhrchen. Die Segmente sind durch brechbare Dichtungen flüssigkeitsdicht voneinander getrennt. In den Segmenten befinden sich verschiedene Reagenzien. In ein solches Röhrchen wird eine Probe eingefüllt. Durch Öffnen einer brechbaren Dichtung wird die eingefühllte Probe mit einem Reagenz in Verbindung gebracht und entsprechend behandelt.

Die Druckschrift WO 03/007677 offenbart, eine Vorrichtung mit einem flexiblen, vollständig abgedichteten Behälter, mit der eine flüssige Probe in dem flexiblen Behälter auf unterschiedliche Temperaturen gebracht werden kann, umso eine PCR durchführen zu können. Mithilfe von bewegbaren Heizelementen wird die Flüssigkeit in dem flexiblen Behälter durch Zusammenpressen von Abschnitten in gewünschte Temperaturzonen hinein bewegt.

Aufgabe der Erfindung ist es, das Verfahren einfacher und verbessert auszugestalten. Die Aufgabe der Erfindung wird mit einem Verfahren mit den Merkmalen des ersten Anspruchs gelöst.

Zur Lösung der Aufgabe wird ein flexibler Schlauch bereit gestellt, der während der Durchführung der Probenpräparation und/ oder Amplifikation unten und damit on einer Seite stets verschlossen ist. In diesem Schlauch wird eine biologische Probe präpariert und/ oder amplifizlert. Lediglich einen Schlauch einzusetzen, ist sehr preiswert. Der Schlauch ist ein Wegweifartikel, der nach einer Präparation oder Amplifikation nebst Inhalt umweltgerecht entsorgt werden korn. Da im übrigen der Schlauch lediglich mit Probe. Substanzen und Reagenzien zu gegebener Zeit zu befüllen und zu hondhaben ist, kann das Verfahren einfach und preiswert auch automatisiert durchgeführt werden.

Der Schlauch ist im Unterschied zu dem aus der Druckschrift US 2004/0161788 A1 bekannten Stand der Technik nicht in Segmente unterteilt, die untereinander zunächst abgedichtet sind. Auch ist es nicht erforderlich, den Schlauch zunächst mit Reagenzien oder Substanzen vorzutüllen.

In den Schlauch wird in einer Ausführungsform der Erfindung in einem ersten Schritt eine Probe eingefüllt, so zum Beispiel hinein pipettiert. Anschließend wird der Schlauch oberhalb der eingefüllten Probe dient zusammengedrückt und so die im Schlauch befindliche Probe von der Umwelt abgeschlossen. Im Anschluss daran wird bei den nachfolgenden Schritten der Schlauch so gehandhabt, dass der in den Schlauch bereits eingefüllte Inhalt gegenüber der Umwelt stets abgeschlossen ist. Werden Reagenzien oder andere zur Durchführung des Verfahrens benötigte Substanzen zugegeben, so geschieht dies nach einem Schleusenprinzip, welches gewährleistet, dass der übrige Inhalt des Schlauches jederzeit gegenüber der Umwelt abgeschlossen ist, so dass keine Kantaminationen auftreten können.

Das Verfahren kann aber beispielsweise auch damit beginnen, dass zunächst ein erstes Reagenz, insbesondere ein Lysepuffer in den Schlauch gefüllt wird und erst im Anschluss daran die biologische Probe.

Um eine Substanz bzw. ein Reagenz einzuschleusen, wird von oben die Substanz bzw. das Reagenz in den Schlauch eingefüllt, der oberhalb des weiteren Inhalts dicht zusammengepresst ist. Nach dem Einfüllen wird der Schlauch oberhalb der eingefüllten Substanz bzw. oberhalb des eingefüllten Reagenz dicht zusammengedrückt. Anschließend wird der darunter befindliche zusammengepresste Teil des Schlauchs entlastet und so geöffnet, so dass die Substanz bzw. das Reagenz schwerkraftbedingt nach unten weiter in den Schlauch in den gewünschten Bereich hinein transportiert wird. Da der Transport durch Schwerkraft bewirkt wird, muss kein Überdruck im Schlauch erzeugt werden.

Zur Durchführung des Verfahrens genügt es, Reagenzien oder andere Substanzen zuzuführen. Eine eingesetzte Robotik wie ein Pipettierroboter muss lediglich Reagenzien oder Substanzen geeignet in den Schlauch einfüllen und zwar vorzugsweise hinein pipettieren. Ein Schlauch wird dafür beispielsweise stationär befestigt. Mit Hilfe einer an für sich bekannten Robotik werden aus Vorratsgefäßen die jeweils gewünschten Reagenzien oder Substanzen entnommen und in den Schlauch zu gegebener Zeit hinein pipettiert.

Das Verfahren zur Durchführung einer Probenpräparation und Amplifikation setzt lediglich voraus, dass Schlauchbereiche durch Zusammendrücken und Entlasten geöffnet und geschlossen werden können, mit Hilfe eines Magneten zu gegebener Zeit separiert wird sowie das zur Durchführung einer Amplifikation geheizt und gekühlt werden kann, wie den nachfolgenden Ausführungen zu entnehmen ist. Reagenzien und Substanzen müssen nicht zwingend nach dem Schleusenprinzip eingefüllt werden, um zu guten Ergebnissen zu gelangen. Das Schleusenprinzip ist allerdings vorteilhaft, um Kontaminationen zu vermeiden.

Der Schlauch besteht vorzugsweise aus einem geeignet inerten Material wie Silikon. Darüber hinaus wird die Innenwand des Schlauchs bevorzugt so behandelt, dass Beads leichter die Innenwand entlang gleiten können. Zu diesem Zweck wird die Schlauchinnenwand beispielsweise silanisiert.

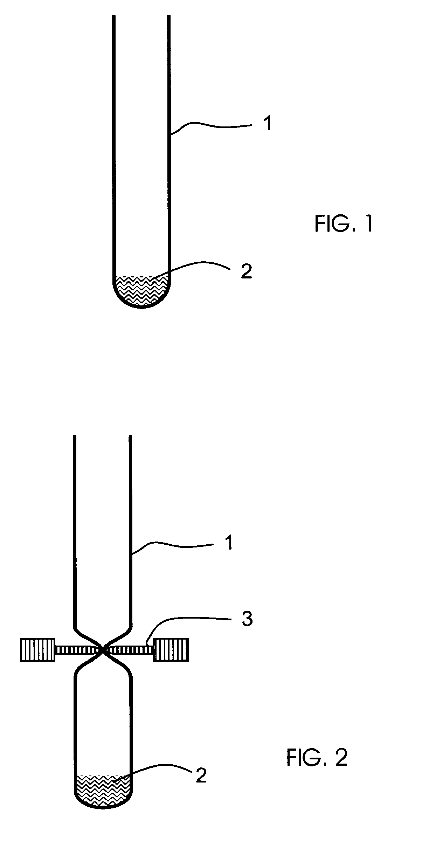

Figur 1 zeigt im Schnitt einen Schlauch 1 , der unten geschlossen ist. In diesen Schlauch 1 wurde eine flüssige biologische Probe 2 eingefüllt. Oberhalb der Probe wird anschließend der Schlauch durch Pressmittel 3 zusammengepresst, wie Figur 2 verdeutlicht. Die Probe 2 ist nun gegenüber der Umwelt abgeschlossen und kann diese nicht kontaminieren.

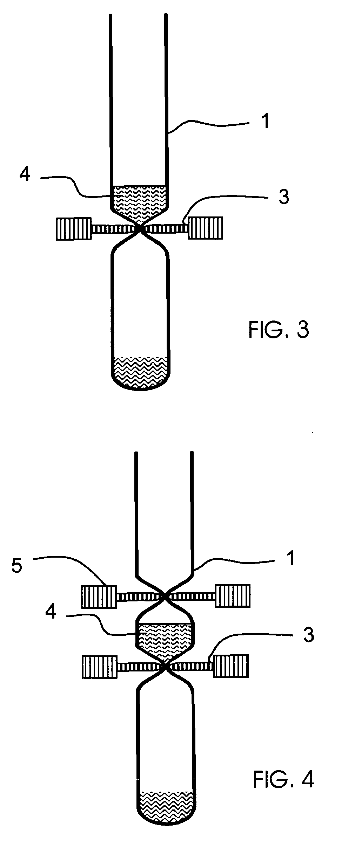

Nach dem Zusammenpressen wird ein Lysepuffer 4 in den Schlauch eingefüllt, wie in Figur 3 gezeigt wird. Im Anschluss an das Einfüllen des Lysepuffers wird der Schlauch oberhalb des Puffers durch Pressmittel 5 verschlossen, wie die Figur 4 verdeutlicht. Die Figuren 3 bis 5 veranschaulichen das Schleusenprinzip, wie also ein Reagenz oder eine Substanz in einen gewünschten Bereich des Schlauchs 1 hinein gebracht wird, ohne dass der sonstige Inhalt des Schlauchs in die Umwelt hinein gelangen kann.

Um die Lyse zu unterstützen, wird der untere Bereich des Schlauches in einer Ausführungsform der Erfindung beheizt und zwar vorzugsweise auf Temperaturen zwischen 37°C und 70°C.

In einer weiteren Ausführungsform der Erfindung wird der Bereich des Schlauches, in dem sich die Probe 2 und der Lysepuffer 4 befindet, beispielsweise durch die Pressmittel 3 abwechselnd teilweise zusammengedrückt und wieder entspannt, um so den Lysepuffer mit der Probe zu vermischen. Es ist dabei nicht erforderlich und auch nicht gewünscht, den Schlauch dicht zusammenzupressen, da nicht abgedichtet werden soll, sondern lediglich Turbulenzen in den beiden Flüssigkeiten zwecks Vermischen erzeugt werden sollen.

In einer Ausführungsform der Erfindung weist der eingesetzte Lysepuffer einen Salzgehalt von insgesamt weniger als 50 Millimolar auf. Es hat sich herausgestellt, dass die Beachtung dieser Grenze sich günstig auf die PCR-Sensitivität auswirken kann. Diese Ausführungsform ist vor allem dann von Vorteil, wenn eine Elution im Rahmen einer Probenvorbereitung nicht durchgeführt wird.

Der pH-Wert des verwendeten Lysepuffers ist vorzugsweise auf Werte zwischen pH 7,5 bis 9,5 eingestellt. Dies steigert die PCR-Sensitivität, wenn an Beads amplifiziert wird. Um den pH-Wert in genannter Weise zu stabilisieren, werden vorzugsweise kommerziell erhältliche Puffer Tris, Hepes und/ oder Mops insbesondere mit einer Konzentration von 10 bis 20 Millimolar eingesetzt, um zu guten PCR-Sensitivitäten zu gelangen. Die vorgenannten Bezeichnungen stellen Abkürzungen dar, die dem Fachmann geläufig sind.

Als Detergenzien werden für die Durchführung der Lyse in einer Ausführungsform der Erfindung nichtionische eingesetzt. Geeignete nichtionische Detergenzien werden unter den Markenbezeichnungen Tween®, Nonidet P-40, Triton X-1 00 oder Brij® kommerziell vertrieben. Auch diese Auswahl ermöglicht es, zu guten PCR-Sensitivitäten zu gelangen.

Die Konzentration von nichtionischen Detergenzien beträgt vorzugsweise 0,1 bis 0,4 Vol.-%.

Optional wird zum Lysepuffer Polyvinylpyrrolidon (abgekürzt als: PVP) und zwar insbesondere von 0,05 bis zu 0,1 Vol.-% hinzugefügt. Durch PVP werden Inhibitoren gebunden und so weiter verbessert eine gute PCR-Sensitivität erhalten.

Im Anschluss an die Lyse werden weitere Substanzen zugeführt, und zwar insbesondere zunächst magnetische Partikel, die magnetische Beads genannt werden, an die die durch die Lyse freigesetzte Nukleinsäure bindet. Darüber hinaus können Substanzen hinzugefügt werden, die diese Bindung der Nukleinsäure an die Beads unterstützen. Dies geschieht stets nach dem Schleusenprinzip. Es werden also zunächst die entsprechenden Reagenzien oder Substanzen in den Schlauch hinein pipettiert, der oberhalb seines Inhalts dicht zusammengepresst ist. Anschließend wird oberhalb der zuletzt hinzugefügten Substanz bzw. oberhalb des neu hinzugefügten Reagenz der Schlauch dicht zusammengepresst. Anschließend wird der unterhalb der zuletzt hinzugefügten Substanz bzw. oberhalb des neu hinzugefügten Reagenz verschlossene Schlauchbereich entlastet, um so die gewünschten Substanzen und Reagenzien in den darunter befindlichen Bereich zuzuführen.

Als Beads, an die Nukleinsäure bindet, werden vorzugsweise magnetische Silica Beads eingesetzt, die zum Beispiel aus der Druckschrift EP 1 337 541 B1 bekannt sind. Neben den bevorzugten magnetischen Silica Beads können jedoch auch alternativ Magnetpartikel mit Ionenaustauscheroberflächen, insbesondere mit einer Anionenaustauschoberfläche eingesetzt werden, um die freigesetzte Nukleinsäure an magnetische Partikel zu binden. Nach dem Stand der Technik ist zwar das Binden mittels Anionenaustausch mit dem Nachteil verknüpft, dass die Elution den Einsatz von Salzen mit hoher Konzentration erfordert, was im Anschluss an die Elution eine Entsalzung erforderlich macht. Entfällt allerdings die Elution, so entfällt auch eine nachfolgende Entsalzung.

Magnetische Beads können sofort in den Schlauch eingefüllt werden, bevor Probe oder Lysepuffer hinzugefügt wird. Diese Ausführungsform der Erfindung trägt dazu bei, dass das Verfahren schnell durchgeführt werden kann.

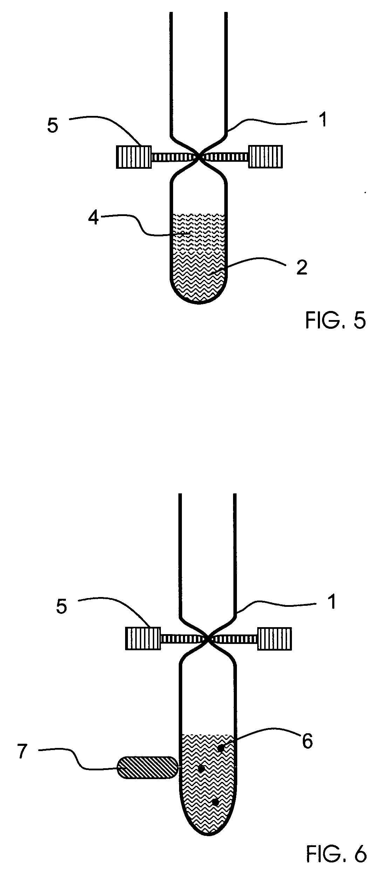

Figur 6 zeigt die Situation, dass Nukleinsäure an magnetische Partikel 7 gebunden worden sind und sich im unteren Bereich des Schlauches innerhalb der dort befindlichen Flüssigkeit befinden. Es wird nun ein Magnet 7 an den Schlauch 1 herangefahren, um so die magnetischen Partikel 6 einzusammeln und nach oben transportieren zu können. Der Schlauch wird daher vorzugsweise durch den Magneten eingedrückt, um den Abstand zwischen dem Magneten 7 und den Partikeln 6 zu verkürzen und so zu gewährleisten, dass die magnetischen Partikel vollständig eingesammelt werden. Aus gleichem Grund wird der Magnet 7 geeignet hoch und herunter angrenzend an den Bereich mit der darin befindlichen Flüssigkeit bewegt.

Sind schließlich so die magnetischen Partikel wie in Figur 7 skizziert eingesammelt worden, so wird der Magnet angrenzend an den Schlauch nach oben bewegt, wie die Figur 8 verdeutlicht. Unterhalb der so aus der Flüssigkeit heraus transportierten magnetischen Partikel wird der Schlauch wiederum durch Zusammenpressen verschlossen. Die nun nicht mehr benötigten Reagenzien verbleiben nach außen abgeschlossen im Schlauch und können nicht in schädlicher Weise in die Umgebung entweichen. Dieser untere abgeschlossene Bereich dient also als Kammer für die Aufnahme von Abfallprodukten des Verfahrens. Darüber befindet sich eine abgeschlossene Kammer bzw. ein abgeschlossener Bereich, in der sich die magnetischen Partikel mit den daran gebundenen Nukleinsäuren befinden, wie die Figur 9 zeigt.

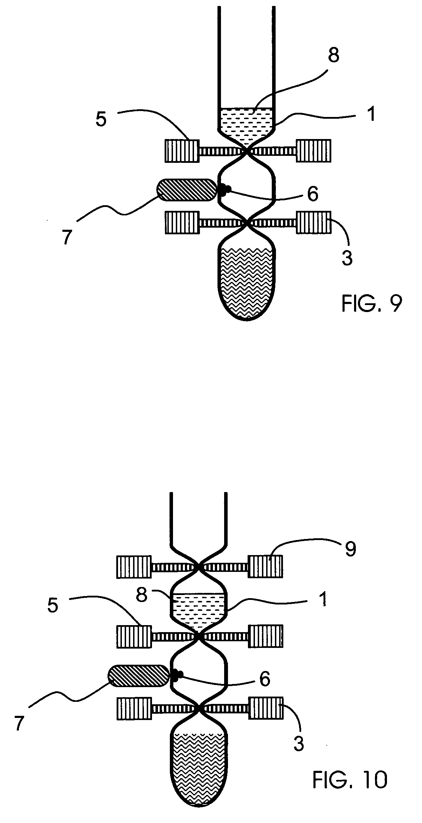

Im Anschluss daran werden Waschpuffer 8 in die Kammer mit den darin befindlichen magnetischen Partikeln nach dem Schleusenprinzip hinzugefügt. Zunächst wird ein Waschpuffer eingefüllt, wie in Figur 9 gezeigt wird. Der Waschpuffer befindet sich dann oberhalb sowie flüssigkeitsdicht getrennt von der Kammer mit den darin befindlichen magnetischen Partikeln 6. Nach dem Einfüllen des Waschpuffers 8 wird der Schlauch mit Hilfe von Pressmitteln 9 oberhalb des eingefüllten Waschpuffers 8 verschlossen, wie in Figur 10 dargestellt wird. Die darunter befindlichen Pressmittel 5 werden gelöst, so dass der Schlauch entspannt und der Waschpuffer 8 aufgrund der Schwerkraft in den Bereich mit den darin befindlichen magnetischen Partikeln 6 gelangt. Auch dieser Bereich des Schlauches wird in einer Ausführungsform der Erfindung beispielsweise mit Hilfe der Pressmittel 5 zusammengedrückt und wieder entspannt, um so die Durchmischung der Puffer mit den magnetischen Partikeln zu fördern. Vorteilhaft wird der Magnet 7 während des Waschens von dem Schlauch wegbewegt, damit die Partikel sich leicht im Waschpuffer verteilen können. Nach dem Waschen wird der Magnet wieder herangefahren, um die magnetischen Partikel wieder einzusammeln. Um das Einsammeln zu beschleunigen, wird der Schlauch im entsprechenden Bereich beispielsweise mit Hilfe des Magneten zusammengedrückt und/ oder der Magnet wird entlang des relevanten Bereichs nach oben und nach unten verfahren. Der Schlauch kann auch zusammen mit den entsprechenden Pressmitteln um seine Längsachse gedreht werden, um so ein vollständiges Einsammeln der magnetischen Partikel 6 zu gewährleisten. Umgekehrt kann der Magnet um den Schlauch herum bewegt werden, um dies verbessert u erreichen.

Sind die magnetischen Partikel durch den Magneten eingesammelt worden und werden diese an einer Seitenwand durch den äußeren Magneten 7 gehalten, so kann das Pressmittel 3 geöffnet werden, um den nun nicht mehr benötigten Waschpuffer in den unteren Bereich abfließen zu lassen, indem die Abfälle gesammelt und aufbewahrt werden. Anschließend wird der entsprechende Schlauchbereich durch das Pressmittel 3 wieder verschlossen.

Die an die magnetischen Partikel gebundenen Probenbestandteile können nun sofort amplifiziert werden und zwar bevorzugt durch ein zweistufiges Amplifikationsverfahren wie zum Beispiel einer nested PCR, wenn zuvor keine Elution durchgeführt wurde. Es hat sich herausgestellt, dass im Fall einer zweistufigen Amplifikation nach dem Waschen eine Elution nicht zwingend zuvor durchgeführt werden muss, um zu guten Ergebnissen zu gelangen.

Um die Amplifikation durchzuführen, können die magnetischen Partikel aus dem Schlauch in eine separate Kammer für die Durchführung einer Amplifikation gebracht werden.

In einer Ausführungsform wird nach dem Waschen zunächst eine Elution durchgeführt. Dazu werden die geeignete Elutionspuffer mittels Schleusen prinzip den magnetischen Partikeln mit den daran gebundenen Nukleinsäuren zugeführt. Vorteilhaft wird zwecks Durchführung der Elution der Magnet 7 vom Schlauch entfernt, damit sich die magnetischen Partikel problemlos im Elutionspuffer verteilen können. Durch die Elution wird die Nukleinsäure von den magnetischen Beads gelöst. Wurden Silica Beads für das Binden benutzt, so wird vorzugsweise ein Niedrigsalz als Elutionspuffer eingesetzt. Die Elution kann wiederum beschleunigt werden, indem der entsprechende Bereich des Schlauches zusammengedrückt und entspannt wird, um so im Elutionspuffer Turbulenzen zu erzeugen.

Nach Durchführung der Elution ist es zweckmäßig, die magnetischen Beads zu separieren. Dies geschieht beispielsweise mit Hilfe des Magneten 7 in bereits beschriebener Weise.

Sollte nur eine Probenpräparation durchgeführt werden, so ist diese spätestens nach Abschluss der Elution beendet. Der Elutionspuffer mit der darin befindlichen Nukleinsäure kann nun aus dem Schlauch entnommen und in gewünschter Weise weiter verwendet werden. Dabei wird insbesondere der untere Schlauchbereich mit den darin befindlichen Abfällen geschlossen gehalten. Wurde der Elutionspuffer entnommen, so kann der Schlauch beispielsweise nun mit einer Klemme in einem oberen Bereich verschlossen werden. Nun können Pressmittel entspannt und der Schlauch nebst Inhalt und Klemme geeignet entsorgt werden, ohne dass die Gefahr besteht, die Umwelt zu kontaminieren.

Es ist allerdings auch möglich, nach Abschluss der Probenpräparation im Schlauch eine Amplifikation, so beispielsweise eine PCR durchzuführen. In diesem Fall ist der Schlauch zunächst geschlossen zu halten.

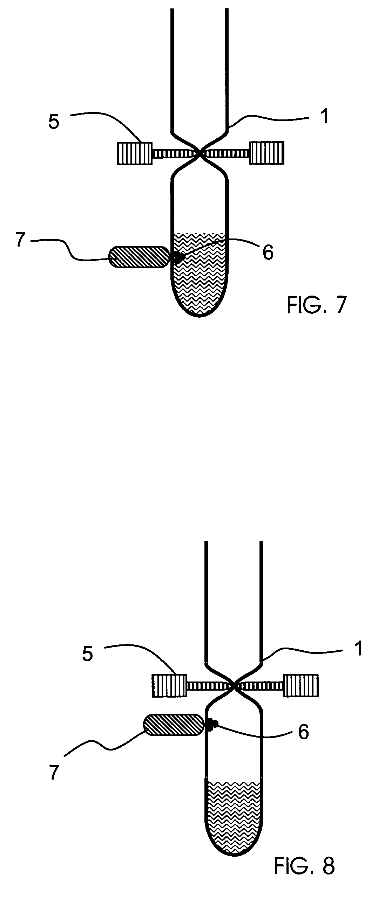

Das bisher anhand der Figuren 1 bis 10 erläuterte Ausführungsbeispiel erfordert einen relativ langen Schlauch und eine Anordnung von Pressmitteln entlang dieses Schlauches. Um die Pressmittel, den Magneten sowie eventuelle Heizmittel auf einem relativ kleinen Bereich anordnen zu können, wird die nachfolgend beschriebene Ausführungsform bevorzugt.

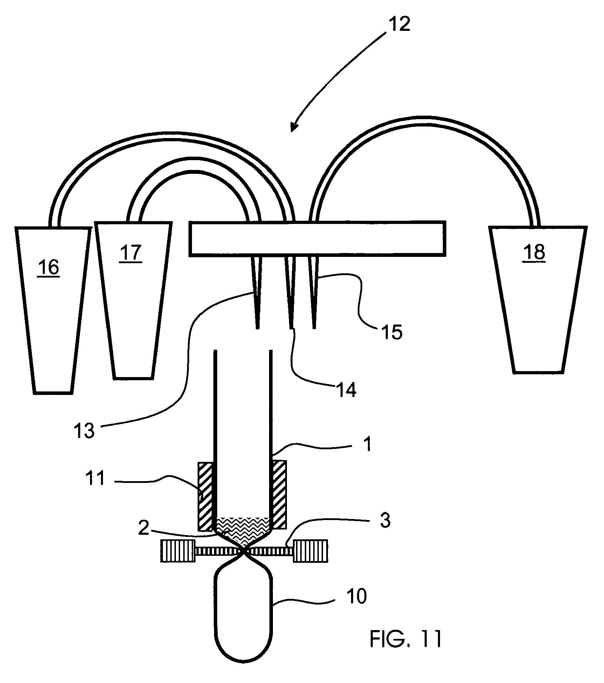

Es wird zunächst ein unterer Bereich des Schlauches durch Zusammendrücken des Schlauches verschlossen. Danach wird die Probe in den Schlauch eingefüllt. Die Probe gelangt nun nicht bis zum Grund des Schlauches, sondern lediglich bis zum Grund des Bereichs, der sich über dem zusammengepressten Teil des Schlauches 1 befindet, wie die Figur 11 verdeutlicht. Im Anschluss daran werden die gewünschten Schritte zur Durchführung einer Präparation und/ oder Amplifikation wie bereits beschrieben durchgeführt. Im Unterschied zu der vorhergehenden Ausführungsform findet jedoch eine Trennung von Lysepuffer und magnetischen Beads nicht statt, indem die magnetischen Beads zu gegebener Zeit mit Hilfe eines Magneten nach oben transportiert werden. Statt dessen werden die Beads durch einen Magneten 7 lediglich an einer Seitenwand des Schlauchs 1 oberhalb eines unteren Bereichs 10 gehalten. Der untere Bereich 10 ist zunächst durch ein durch Pressmittel 3 dicht zusammengepresstes Teilstück des Schlauches von darüber liegenden Schlauchbereichen getrennt. Anschließend werden die Pressmittel 3 geöffnet und der nicht mehr benötigte Lysepuffer fließt nach unten ab. Nachdem der Lysepuffer so von den magnetischen Beads separiert wurde, wird der untere Bereich wieder durch die Pressmittel 3 verschlossen und das Verfahren in beschriebener Weise fortgesetzt.

In Figur 11 werden an einen oberen Bereich des Schlauches angrenzende Kühl- und Heizmittel gezeigt, die elektrisch zu kühlen und heizen vermögen. Mit den Kühl- und Heizmitteln 11 kann der angrenzende Bereich des Schlauches zusammengedrückt und wieder entspannt werden, um so beispielsweise eine Lyse oder eine Amplifikation auch auf mechanische Weise zu beschleunigen. Die Kühl- und Heizmittel 11 sind so dimensioniert und gestaltet, dass eine große Wärmeübertragungsfläche vorhanden ist.

Der Schlauch ist innerhalb einer Vorrichtung untergebracht, die einen Pipettierautomaten 12 umfasst. Der Pipettierautomat weist eine Mehrzahl von verfahrbaren Injektionsnadeln 13, 14 und 15 auf, über die benötigte Flüssigkeiten in das Röhrchen insbesondere automatisiert hinein geleitet werden. Jede Injektionsnadel ist mit einem Vorratsgefäß 16, 17 und 18 verbunden, in denen sich die benötigten Reagenzien und Substanzen befinden, um die Probenpräparation und/ oder Amplifikation durchführen zu können.

In einer Ausführungsform der Erfindung kann ein Ölfilm vorgesehen werden, um verbessert sicherzustellen, dass bei fehlerhafter Handhabung oder anderen Störungen keine Kontamination auftritt. Der auf Puffern schwimmende Ölfilm wirkt dann als zusätzliche Dichtung bzw. Verschluss, um im Behälter befindliche Substanzen und Reagenzien nicht nach außen gelangen zu lassen.

Soll im Anschluss an die Probenpräparation im Schlauch eine Amplifikation durchgeführt werden, so wird in den Schlauch ein PCR-Puffer nach dem Schleusenprinzip eingefüllt und so dem Elutionspuffer mit der darin befindlichen Probe hinzugefügt.

Falls die magnetischen Beads eine Amplifikation behindern sollten, können diese mit Hilfe eines Magneten nach dem Schleusenprinzip aus dem Schlauch nach oben entnommen werden. Vorzugsweise werden jedoch - um jedes Kontaminationsrisiko zu vermeiden - die magnetischen Beads in solchen Fällen am Grund des entsprechenden Bereichs gesammelt und der Schlauch anschließend unmittelbar darüber dicht zusammengepresst. Anschließend wird der unterhalb der magnetischen Beads zusammengepresste Bereich des Schlauchs entspannt und so geöffnet, um dann die magnetischen Beads beispielsweise durch Entfernen des Magneten in den Abfallbereich fallen zu lassen. Zwar geht bei dieser Ausführungsform ein kleiner Teil an bereits präparierter Probe verloren. Dafür wird eine Kontamination verbessert im Vergleich zu dem Fall verhindert, dass die magnetischen Beads aus dem Schlauch entfernt werden.

In der Regel verbleiben die magnetischen Beads allerdings in dem Bereich des Schlauches, in dem die PCR-durchgeführt wird, da die Anwesenheit von magnetischen Beads die Amplifikation grundsätzlich nicht behindert.

Während der Amplifikation wird vorteilhaft der Schlauch beispielsweise durch Heiz- und Kühlmittel 11 so zusammengepresst, dass hierdurch die Wärmeübertragungsfläche vergrößert wird. Als Heiz- und Kühlmittel werden vorzugsweise elektrothermische Wandler (Peltier-Elemente) eingesetzt, um so unproblematisch sowohl Heizen und Kühlen zu können. Diese Ausführungsform ist vorteilhaft, da bei einer PCR Wärme schnell zu- sowie abzuführen ist, um zu guten Resultaten zu gelangen.

Zugleich kann während der Amplifikation der entsprechende Schlauchbereich mehrfach zusammengedrückt und expandiert werden, um so Turbulenzen zu erzeugen und damit den Wärmeaustausch zu fördern. Vorzugsweise werden bei dieser Ausführungsform wiederum die Kühl- und Heizmittel verwendet, um den Schlauchbereich entsprechend zu verformen.

Im Schlauch kann auch eine Realtime-PCR durchgeführt werden. Zu diesem Zweck weist der Schlauch im entsprechenden Bereich ein Sichtfenster auf oder der Schlauch ist vollständig durchsichtig. Während der Amplifikation werden mit Hilfe einer Optik Fluoreszenzänderungen ermittelt, um so in bekannter Weise eine Realtime-PCR durchzuführen.

Eine Amplifikation einer biologischen Probe kann auch vorteilhaft in einem flexiblen, anders geformten Behälter durchgeführt werden, der vor oder während der Amplifikation ein oder mehrfach zusammengedrückt wird und zwar vorzugsweise mit Hilfe von Heiz- und/ oder Kühlmitteln, um so die Amplifikation zu beschleunigen. Der Behälter muss also nicht notwendig ein an einem Ende verschlossener Schlauch sein, um die Amplifikation durch das ein- oder mehrfache Zusammendrücken nebst zwischenzeitlicher Entspannung aus zuvor genannten Gründen zu fördern.

Experimentell wurden u. a. folgende Versuche durchgeführt.

Es wurde ein silanisierter aus Silikon bestehender Schlauch mit 1cm InnenDurchmesser eingesetzt. Durch die Silanisierung gleiten magnetische Beads besonders leicht entlang der Innenwand des Schlauches entlang, was den Transport der magnetischen Beads mit Hilfe eines externen Magneten erleichtert. Zum Silanisieren wurde eine 2-5% w/v Dimethyldichlorsilan-Lösung in einem geeigneten organischen Lösungsmittel (z.B. Cyclotetrasiloxan) benutzt. Silanisierungslösungen findet der Fachmann unter der CAS Nummer: "17-1332-01". Zum Silanisieren wurde ein ca. 12 cm langes Schlauchstück mit Hilfe einer Schlauchklemme an einem Ende verschlossen und mit der Lösung befüllt. Die Lösung kann sofort wieder heraus gegossen werden. Nach Lösen der Schlauchklemme wurde der Schlauch für eine Stunde in einem Abzug getrocknet.

### Versuch A / Nucleic Isolation Process A:

Bei diesem Ansatz wurde das untere Schlauchende dauerhaft mit einer Schlauchklemme verschlossen. Eine weitere Schlauchklemme wurde zusätzlich benötigt, um nach jedem Flüssigkeitswechsel und dem "Herausschieben" der magnetischen Silicabeads aus der Flüssigkeit durch das Abklemmen eine neue Präparationszone einzurichten. Dieser Vorgang wird mehrfach wiederholt. Das "Herausschieben" der magnetischen Silicabeads aus der Flüssigkeit wurde mit Hilfe eines starken Permanentmagnet durchgeführt.

### Praktische Durchführung:

In den Schlauch wurde 200µl Blut und 200µl Buffer AL und 20µl ProteinaseK pipettiert. Danach wurde der Schlauch 15 min in ein auf 56°C temperiertes Wasserbad gehängt. Nach der Inkubation wurde 200µl isopropanol und 15 µl MagAttract Suspension G zugesetzt. Durch mehrfaches (1 0-20x) Zusammendrücken und Relaxieren des Schlauches wurde der Inhalt vermischt. Für einen Zeitraum von 3 min wurde das Vermischen alle 20 sec wiederholt. Nun wurde von Außen ein starker Permanentmagnet an die Flüssigkeit angesetzt und die magnetischen Silicabeads für 20 sec magnetisch sedimentiert. Langsam wurde der Magnet um 2-3 cm nach oben geschoben, bis dieser sich außerhalb der Flüssigkeit befand. Nun wurde mit einer zweiten Schlauchklemme der Bereich zwischen Flüssigkeitsmeniskus und der Position des Magnets dicht zusammengepresst. Damit wurde nun eine neue Reaktionszone eingerichtet. Nach Entfernen des Permanentmagnets wurde 1 ml Waschpuffer Buffer AW1 von oben in den Schlauch gefüllt, Durch mehrfaches (10-20x) Zusammendrücken und Relaxieren des Schlauches wurden die Magnetpartikel wieder resuspendiert. Nun wurde wieder von Außen der Permanentmagnet an die Flüssigkeit angesetzt und die magnetischen Silicabeads für 20 sec magnetisch sedimentiert. Langsam wurde erneut der Magnet um 2-3 cm nach oben geschoben, bis sich die magnetischen Partikel außerhalb der Flüssigkeit befanden. Mit einer dritten Schlauchklemme, die zwischen den Flüssigkeitsmeniskus und der Position des Magnets gesetzt wurde, wurde eine nächst höher-gelegene Reaktionszone eingerichtet. Der Waschvorgang wurde ein weiteres Mal mit dem Waschpuffer Buffer AW2 wiederholt. Das Abklemmen einer neuen Reaktionszone konnte diesmal durch Lösen und Setzen der "zweiten" Schlauchklemme erfolgen. Zur Elution befanden sich die Magnetpartikel nun in einer neuen Reaktionszone und weitest gehend waren Flüssigkeitsreste des Waschpuffers entfernt. Vor der Elution wurden die magnetischen Beads durch einen leichten Luftstrom 5 min lang getrocknet, um Reste an Ethanol-haltigem Waschpuffer zu entfernen. Nun wurde durch Zugabe von oben mit 200µl RNase-freiem Wasser elutiert. Hierzu wurde für einen Zeitraum von 1 min kontinuierlich der Schlauch zusammengedrückt und wieder entspannt. Dabei wurde das Magnetpartikelsediment resuspendiert und die gebundenen Nukleinsäuren wurden eluiert. Als letzter Schritt wurde von Außen ein starker Permanentmagnet oberhalb der mittleren Schlauchklemme angesetzt und die magnetischen Silicabeads für 20 sec magnetisch sedimentiert. Durch einen Gegendruck (also von der gegenüberliegenden Seite) mittels eines weiteren Magneten oder geeigneten Gegenstandes wurde das Magnetsediment eingequetscht und die Flüssigkeit (Eluat) in eine neue darüber liegende Reaktionszone oder auch Entnahmezone transferiert. Dieses Magnetpartikel-befreite Eluat wurde nun entnommen und analysiert. Die in diesem Absatz verwendeten Handelsnamen sind Marken oder Handelsnamen der Firma QIAGEN GmbH aus Hilden, Deutschland. Die Produkte werden unter diesen Bezeichnungen von der Fa. QIAGEN GmbH kommerziell vertrieben.

### Versuch B / Nucleic Isolation Process B)

Bei diesem Ansatz wurde das untere Schlauchende dauerhaft mit einer Schlauchklemme verschlossen. Eine weitere Schlauchklemme wurde in der Schlauchmitte platziert. Oberhalb der oberen Klemme befand sich die Präparationszone. Für den Flüssigkeitswechsel wurden die magnetischen Silicabeads mit Hilfe eines starken Permanentmagnet festgehalten. Durch das Lösen der mittleren Schlauchklemme lief die Flüssigkeit einfach in den unteren Bereich.

### Praktische Durchführung:

Der obere Schlauchteil wurde mit 200µl Blut und 200µl Buffer AL und 20µl ProteinaseK befüllt und 15 min in ein auf 56°C temperiertes Wasserbad gehängt. Nach der Inkubation wurde 200µl Isopropanol und 15 µl MagAttract Suspension G zugesetzt. Durch mehrfaches (1 0-20x) Zusammendrücken und Relaxieren des Schlauches wurde der Inhalt vermischt. Für einen Zeitraum von 3 min wurde das Vermischen alle 20 sec wiederholt. Nun wurde von Außen ein starker Permanentmagnet oberhalb der mittleren Schlauchklemme angesetzt und die magnetischen Silicabeads für 20 sec magnetisch sedimentiert. Während der Magnet die Silicabeads in Position hielt, wurde die mittlere Schlauchklemme gelöst und die Flüssigkeit lief nach unten in den unteren Bereich ab. Die mittlere Schlauchschelle wurde wieder verschlossen. Nun wurde der Permanentmagnet entfernt und 1 ml Waschpuffer Buffer AW1 von oben in den Schlauch gefüllt und durch mehrfaches Zusammendrücken (ca. 10-20 mal) und Relaxieren des Schlauches wurden die Magnetpartikel wieder resuspendiert. Die Magnetseparation und das Entfernen des Waschpuffers verlief wie zuvor beschrieben. Die Waschprozedur wurde ein weiteres Mal mit dem Waschpuffer Buffer AW2 wiederholt. Nachdem der Waschpuffer in den unteren Bereich geleitet und die mittlere Schlauchklemme geschlossen wurde, wurde der sogenannte "Waterinse" durchgeführt. Dabei bleibt der Permanentmagnet auf Position, und es wird vermieden, das Magnetpartikelsediment zu verwirbeln. Beim Waterrinse wurde vorsichtig 1 ml Wasser von oben in den Schlauch gefüllt. Nach 5 sec wurde die mittlere Schlauchklemme gelöst und das Wasser lief nach unten in den unteren Bereich ab. Der Magnet hielt die Silicabeads während dieser Prozedur die ganze Zeit lang in Position. Die mittlere Schlauchklemme wurde nun wieder geschlossen und die Elution wurde durch Zugabe von oben in den Schlauch mit 200µl RNase-freiem Wasser durchgeführt. Hierzu wurde für einen Zeitraum von 1 min kontinuierlich der Schlauch zusammengedrückt und wieder entspannt. Dabei wurde das Magnetpartikelsediment resuspendiert und die gebundenen Nukleinsäuren wurden eluiert. Als letzter Schritt wurde von Außen ein starker Permanentmagnet oberhalb der mittleren Schlauchklemme angesetzt und die magnetischen Silicabeads für 20 sec magnetisch sedimentiert. Der Überstand wurde nun von oben abpipettiert und enthielt die aufgereinigten Nukleinsäuren, wie eine nachfolgende Analyse ergab.

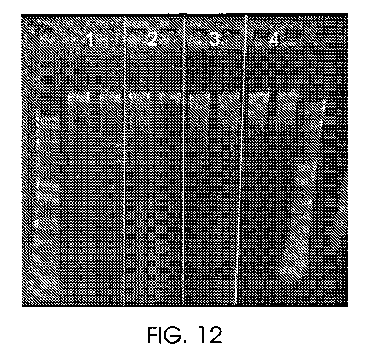

Figur 12 verdeutlicht das Ergebnis einer elektrophoretischen Analyse (0,8% Agarosegel, 8µl Eluat pro Tasche aufgetragen)
1 : Schlauchpräparationen mit "Lower Waste" Strategie (Versuch B)
2: Schlauchpräparationen mit "Moving Magnets" Strategie (Versuch A)
3: Referenzpräparation durchgeführt mit Hilfe des QiAamp Blood DNA Mini Kits (der Fa. QIAGEN GmbH aus Hilden)
4: Referenzpräparation durchgeführt mittels des QIAGEN BioSprint 15

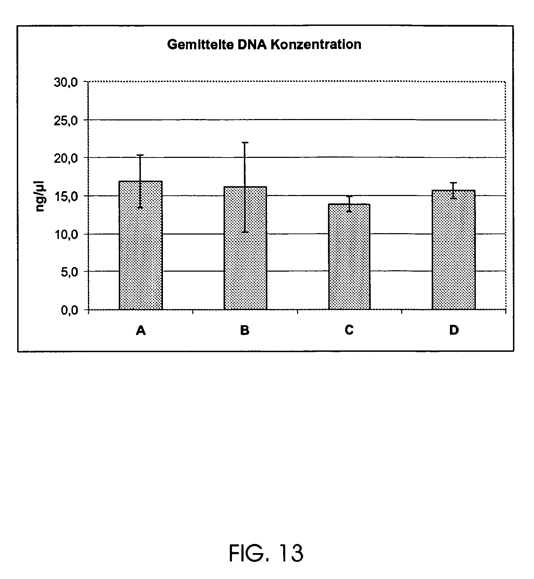

Figur 13 zeigt das Ergebnis der DNA Quantifizierung mittels UV Spektrometrie
A: Schlauchpräparationen mit "Lower Waste" Strategie (Nucleic Isolation Process B)
B: Schlauchpräparationen mit "Moving Magnets" Strategie (Nucleic Isolation Process A)
C: Referenzpräparation durchgeführt mit Hilfe des QIAamp Blood DNA Mini Kits (QIAGEN)
D: Referenzpräparation durchgeführt mittels des QIAGEN BioSprint 15

### Schlussfolgerungen:

Die Figur 1 2 und 13 zeigen, dass die Probenvorbereitung in einem Schlauch vergleichbare Ergebnisse liefert wie Standard-Referenzmethoden. Bedingt durch die neuen Materialien und Abläufe lassen sich neue Strategien der Nukleinsäurepräparation verwirklichen.

## Patentansprüche

1. Verfahren zur Durchführung einer Präparation und/ oder Amplifikation einer biologischen Probe, bei dem eine biologische Probe (2) in einem an einem Ende verschlossenen Schlauch (1) präpariert und/ oder amplifiziert wird, **dadurch gekennzeichnet, dass** nach einem Schleusenprinzip ein Reagenz oder eine andere zur Durchführung des Verfahrens benötigte Substanz zugegeben wird, indem von oben die Substanz bzw. das Reagenz in den Schlauch eingefüllt wird, der oberhalb eines weiteren Inhalts dicht zusammen gepresst ist, nach dem Einfüllen wird der Schlauch oberhalb der eingefügten Substanz bzw. oberhalb des eingefügten Reagenz dicht zusammengedrückt, anschließend wird der darunter befindliche zusammengepresste Teil des Schlauchs entlastet und so geöffnet, so dass die Substanz bzw. das Reagenz schwerkraftbedingt nach unten weiter in den Schlauch in den gewünschten Bereich hinein transportiert wird.

2. Verfahren nach Anspruch 1, bei dem eine biologische Probe (2) in den Schlauch (1) gefüllt wird, im Anschluss daran der Schlauch (1) oberhalb der Probe dicht zusammengepresst wird, anschließend ein Reagenz (4) oder eine Substanz in den Schlauch (1) eingefüllt wird, der Schlauch (1) oberhalb des eingefüllten Reagenz (4) oder der eingefüllten Substanz dicht zusammengepresst wird, anschließend die durch Zusammenpressen geschaffene dichte Verbindung unterhalb des eingefüllten Reagenz (4) oder der eigefüllten Substanz geöffnet wird.

3. Verfahren nach einem der vorhergehenden Ansprüche, bei dem als Reagenz oder Substanz ein Lysepuffer (4), ein Waschpuffer, ein Elutionspuffer, magnetische Partikel (6) für das Binden von Nukleinsäure oder ein Puffer für die Amplifikation von Nukleinsäure für die Durchführung des Verfahrens eingesetzt wird.

4. Verfahren nach einem der vorhergehenden Ansprüche, bei dem an magnetische Partikel (6) gebundene Nukleinsäure einer biologischen Probe von einem Reagenz (4) oder einer Substanz mit Hilfe eines Magneten (7) getrennt wird, mit dem die magnetischen Partikel (6) durch den Magneten (7) eingesammelt und gehalten werden und im Anschluss daran die magnetischen Partikel (6) entweder aus dem Reagenz (4) oder der Substanz mit Hilfe des Magneten herausgezogen werden oder durch Öffnen eines dicht zusammengepressten Bereichs des Schlauchs (1), der sich unterhalb der gehaltenen magnetischen Partikel (6) befindet, das Reagenz (4) oder die Substanz entfernt wird.

5. Verfahren nach einem der vorhergehenden Ansprüche, bei dem nicht mehr benötigte Reagenzien oder Substanzen am Grunde des Schlauches (1) gesammelt werden.

6. Verfahren nach einem der vorhergehenden Ansprüche, bei dem während des Aufschließens, des Waschens, des Eluierens oder des Amplifizierens einer Probe der Schlauch (1) mit Hilfe von Pressmitteln (3, 5, 9), mit Hilfe von Heiz- und/ oder Kühlmitteln oder mit Hilfe eines Magneten (7) ein- oder mehrfach zusammengedrückt und entlastet wird.

7. Verfahren nach einem der vorhergehenden Ansprüche, bei dem der Schlauch (1) keine in durch stoffschlüssige Dichtungen unterteilte Segmente oder Kammern umfasst und/ oder nicht mit Reagenzien oder Substanzen vorgefüllt worden ist.

8. Verfahren nach einem der vorhergehenden Ansprüche, bei dem eine Amplifikation einer biologischen Probe durchgeführt wird und der Behälter vor oder während der Amplifikation ein oder mehrfach zusammengedrückt wird und zwar vorzugsweise mit Hilfe von Heiz- und/ oder Kühlmitteln.

## Claims

1. A process for performing preparation and/or amplification of a biological sample wherein a biological sample (2) is prepared and/or amplified in a tubing (1) which is closed on one end, **characterised in that** a reagent or another substance required for performing the process is added according to the air-lock principle by way of introducing the substance or the reagent, respectively, into the tubing from the top, the tubing is tightly compressed above another content, following introduction the tubing will tightly be compressed above the introduced substance or reagent, respectively, the compressed lower section of the tubing will subsequently be released and will be opened such that the substance or the reagent, respectively, will be moved further downwards into the tubing to the desired section due to gravitational force.

2. The process according to claim 1 wherein a biological sample (2) is introduced into the tubing (1), the tubing (1) subsequently will tightly be compressed above the sample, a reagent (4) or another substance will then be introduced into the tubing (1), the tubing (1) will tightly be compressed above the introduced reagent (4) or the introduced substance, the tight interconnection generated by the compression will then be opened below the introduced reagent (4) or the introduced substance.

3. The process according to one of the preceding claims, wherein a lysis buffer (4), a washing buffer, an elution buffer, magnetic particles (6) for binding of nucleic acid or a buffer for amplification of nucleic acid is employed as a reagent or substance for performing the process.

4. The process according to one of the preceding claims, wherein nucleic acid attached to magnetic particles (6) of a biological sample is separated from a reagent (4) or another substance by way of a magnet (7) by which magnet the magnetic particles (6) are captured by the magnet (7) and will be retained and subsequently the magnetic particles (6) will either be extracted from the reagent (4) or the substance by way of the magnet, or the reagent (4) or the substance will be removed by opening of a tightly compressed section of the tubing (1) located below the retained magnetic particles (6).

5. The process according to one of the preceding claims, wherein the reagents or substances which are no longer required are collected on the bottom of the tubing (1).

6. The process according to one of the preceding claims, wherein during processing, washing, eluting or amplifying of a sample the tubing (1) is compressed and released one or several times by way of compression means (3, 5, 9), by way of heating and/or cooling means or by way of a magnet (7).

7. The process according to one of the preceding claims, wherein the tubing (1) does not comprise segments or compartments which are separated by firmly bonded sealings and/or has not been prefilled with reagents or substances.

8. The process according to one of the preceding claims, wherein an amplification of a biological sample is performed and the container is compressed one or several times during amplification preferably by way of heating and/or cooling means.

## Revendications

1. Procédé pour la mise en oeuvre d'une préparation et/ou amplification d'un échantillon biologique dans lequel un échantillon biologique (2) est préparé and/ou amplifié dans un tuyau (1) qui est fermé sur une extrémité, **caractérisé par le fait qu'**un réactif ou une autre substance qui est nécessaire pour la mise en oeuvre du procédé est ajouté selon le principe de l'écluse par l'introduction de la substance ou du réactif, respectivement, d'en haut dans le tuyau, qui est étanchement comprimé au-dessus de la substance ou du réactif introduit, respectivement, la partie inférieure comprimé du tuyau va ensuite être relâchée et va être ouverte de manière que la substance ou le réactif, respectivement, va être déplacé vers le bas dans le tuyau jusqu'à la partie souhaitée en raison de la gravitation.

2. Procédé selon la revendication 1 dans lequel un échantillon biologique (2) est introduit dans le tuyau (1), le tuyau ensuite est étanchement comprimé au-dessus d'échantillon, et puis un réactif (4) ou un autre substance est introduit dans le tuyau (1), le tuyau est étanchement comprimé au-dessus de la substance ou le réactif introduit, et ensuite la connexion étanche générée par la compression est ouverte au-dessous du réactif ou de la substance introduite (4).

3. Procédé selon l'une des revendications précédentes dans lequel en tant que réactif ou substance un tampon de lyse (4), un tampon de lavage, un tampon d'élution, des particules magnétiques (6) pour lier d'acide nucléique ou un tampon pour l'amplification d'acide nucléique pour la mise en oeuvre du procédé est utilisé.

4. Procédé selon l'une des revendications précédentes dans lequel l'acide nucléique lié aux particules magnétiques (6) d'un échantillon biologique est séparé d'un réactif (4) ou d'une autre substance par un aimant (7), par lequel aimant les particules magnétiques (6) sont capturées et sont retenues et ensuite le particules magnétiques (6) sont extraites soit du réactif soit de la substance par l'aimant, ou le réactif (4) ou la substance sont enlevés par l'ouverture une partie étanchement comprimée du tuyau (1) localisée au-dessous des particules retenues (6).

5. Procédé selon l'une des revendications précédentes dans lequel les réactifs ou les substances qui ne sont plus nécessaires sont recueillis sur le fond du tuyau (1).

6. Procédé selon l'une des revendications précédentes dans lequel, pendent le traitement le lavage, l'élution ou l'amplification d'un échantillon, le tuyau (1) est comprimé et relâché un ou plusieurs fois par des moyens de compression (3, 5, 9), par des moyens de chauffage et/ou de refroidissement ou par un aimant (7).

7. Procédé selon l'une des revendications précédentes dans lequel le tuyau ne comprend pas des parties ou des compartiments qui sont séparés par des joints d'étanchéité permanents et/ou n'a pas été rempli en avant avec des réactifs ou des substances.

8. Procédé selon l'une des revendications précédentes dans lequel une amplification d'un échantillon biologique est effectuée et le récipient est comprimé une ou plusieurs fois pendant l'amplification préférablement par des moyens de chauffage et/ou de refroidissement.
